(19) Europäisches Patentamt
European Patent Office
Office européen des brevets

(11) **EP 1 792 651 A1**

(12) **EUROPÄISCHE PATENTANMELDUNG**

(43) Veröffentlichungstag:
**06.06.2007 Patentblatt 2007/23**

(51) Int Cl.:
*B01J 23/881* (2006.01)        *B01J 35/02* (2006.01)
*B01J 37/02* (2006.01)        *B01J 37/08* (2006.01)
*C07C 45/38* (2006.01)

(21) Anmeldenummer: 05025497.8

(22) Anmeldetag: **23.11.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **SÜD-CHEMIE AG**
**80333 München (DE)**

(72) Erfinder:
• **Dr.Gückel, Christian**
**07652 Paramus**
 **New Jersey (US)**

• **Dr.Wanninger, Klaus**
**83059 Kolbermoor (DE)**
• **Dr.Estenfelder, Marvin**
**85560 Ebersberg (DE)**
• **Fischer, Claudia**
**85622 Feldkirchen (DE)**

(74) Vertreter: **Westendorp, Michael Oliver**
**Patentanwälte**
**Splanemann Reitzner**
**Baronetzky Westendorp**
**Rumfordstrasse 7**
**80469 München (DE)**

(54) **Schalenkatalysator, insbesondere zur Oxidation von Methanol zu Formaldehyd sowie Verfahren zu dessen Herstellung**

(57)      Die Erfindung betrifft einen Beschichtungskatalysator, insbesondere zur Oxidation von Methanol zu Formaldehyd, der auf einem inerten, vorzugsweise im wesentlichen unporösen Trägerkörper mindestens eine Beschichtung aufweist, die vor dem Entfernen der organischen Anteile der Komponenten b) bzw. c) enthält: (a) Oxide oder in die entsprechenden Oxide überführbare Vorläuferverbindungen von Molybdän und Eisen, wobei das molare Verhältnis von Mo:Fe zwischen 1:1 und 5:1 liegt, sowie gegebenenfalls weitere metallische bzw. metalloxidische Komponenten oder in die entsprechenden Oxide überführbare Vorläuferverbindungen, (b) mindestens einen organischen Binder, vorzugsweise eine wässrige Dispersion von Copolymeren, insbesondere ausgewählt aus Vinylacetat/Vinyllaurat, Vinylacetat/ Ethylen, Vinyacetat/Acrylat, Vinylacetat/Maleat, Styrol/ Acrylat oder deren Gemischen, und (c) mindestens eine weitere Komponente ausgewählt aus der Gruppe bestehend aus $SiO_2$-Sol oder dessen Vorläufer, $Al_2O_3$-Sol oder dessen Vorläufer, $ZrO_2$-Sol oder dessen Vorläufer, $TiO_2$-Sol oder dessen Vorläufer, Wasserglas, MgO, Zement, monomeren, oligomeren oder polymeren Silanen, Alkoxysilanen, Aryloxysilanen, Acryloxysilanen, Aminosilanen, Siloxanen oder Silanolen. Beschrieben wird zudem ein Verfahren zur Herstellung des Katalysators und seine bevorzugte Verwendung.

**Figur 1**

**Beschreibung**

Die vorliegende Erfindung betrifft einen Schalenkatalysator, im speziellen einen beschichteten Katalysator (Beschichtungskatalysator), insbesondere zur Oxidation von Methanol zu Formaldehyd, der auf einem inerten, vorzugsweise im wesentlichen unporösen Trägerkörper eine Beschichtung aus einer Zusammensetzung aufweist, die neben einer aktiven Masse eine Haftungsvermittlerzusammensetzung enthält, die mindestens einen organischen Binder und mindestens eine weitere, zumindest teilweise anorganische haftungsvermittelnde Komponente, insbesondere eine Solkomponente umfasst.

Molybdän-Eisen-Katalysatoren zur partiellen Oxidation von Methanol zu Formaldehyd sind schon seit längerer Zeit bekannt.

Die Atomverhältnisse zwischen Molybdän und Eisen können bei diesen bekannten Katalysatoren variieren. Ferner kann ein Teil der aktiven Komponenten teilweise durch Titan, Antimon, Zinn, Nickel, Chrom, Cer, Aluminium, Calcium, Magnesium, Vanadium, Niob, Silber und/oder Mangan ersetzt sein. Derartige zum Beispiel Titan enthaltende Katalysatoren sind beispielsweise aus US 3,978,136 für die partielle Oxidation von Methanol zu Formaldehyd bekannt. Hierbei handelt es sich aber im Gegensatz zu dem erfindungsgemäßen Katalysator nicht um einen gecoateten Katalysator.

Unter gecoateten (beschichteten) Katalysatoren, auch Coat- oder Beschichtungskatalysatoren genannt, versteht man Katalysatoren, die durch Beschichten eines (unporösen) Trägerkörpers mit einer porösen Schicht der eigentlichen aktiven Masse entstehen.

Im Unterschied dazu werden bei den Imprägnierverfahren die katalytisch aktiven Zentren (häufig Edelmetalle, wie Pd, Pt, Au, Ag etc.) als Lösung dispers auf einen porösen Träger (häufig: $SiO_2$, $Al_2O_3$, $TiO_2$, $ZrO_2$, Carbon, $Nb_2O_5$ etc.) aufgebracht. Bei den durch das Imprägnierverfahren hergestellten Katalysatoren bestehen zumeist chemischphysikalische Wechselwirkungen zwischen Träger und aktiven Zentren, die entscheidenden Einfluss auf das katalytische Geschehen nehmen. Hingegen dient bei den Coatkatalysatoren der Trägerkörper lediglich der Formgebung ("structural support"). Im Gegensatz zu imprägnierten Katalysatoren, bei denen die aktiven Elemente dispers in dem porösen Träger - gegebenenfalls auch in einer im Träger befindlichen äußeren Schale (= Schalenkatalysator) - verteilt sind, ist beim Coatkatalysator der (unporöse bzw. im wesentlichen unporöse) Trägerkörper von der aktiven Masse umhüllt.

In der US 3,975,302 wird ein nach dem Imprägnierverfahren hergestellter Fe/Mo-Katalysator für die Oxidation von Methanol zu Formaldehyd beschrieben. Demnach werden Eisen und Molybdän als $MoO_4^{2-}$ und $Fe^{3+}$-Salze in Wasser gelöst und dann auf einen porösen Träger mit einer BET-Oberfläche von 1 bis 20 $m^2/g$ imprägniert.

Auch in der US 4,181,629 wird ein Katalysator für die Oxidation von Methanol zu Formaldehyd beschrieben, der durch Imprägnieren im Fliessbettverfahren hergestellt wird.

Die EP-A-0 068 192 betrifft einen abriebfesten Schalenkatalysator, wobei als bevorzugtes Bindemittel in der Schalenzusammensetzung Glucose- oder Harnstoff in wässriger Lösung angegeben sind.

Die EP-A-0 294 775 betrifft ein Verfahren zur Herstellung eines Schalenkatalysators durch Aufsprühen des katalytisch aktiven Materials auf bewegte Trägerteilchen bei Temperaturen von 100 bis 600 °C.

Es besteht jedoch ein Bedarf an verbesserten Katalysatoren zur Oxidation von Methanol zur Formaldehyd, die neben einer sehr guten Abriebfestigkeit auch eine gute Aktivität und Selektivität aufweisen. Aufgabe der vorliegenden Erfindung war es daher, verbesserte Katalysatoren, insbesondere zur Oxidation von Methanol zu Formaldehyd bereitzustellen, die eine besonders hohe Abriebfestigkeit aufweisen und die Nachteile des Standes der Technik vermeiden.

Diese Aufgabe wird gemäß Anspruch 1 gelöst durch einen Schalen- bzw. Beschichtungskatalysator, insbesondere zur Oxidation von Methanol zu Formaldehyd, der auf einem inerten, vorzugsweise im wesentlichen unporösen Trägerkörper mindestens eine Beschichtung aufweist, die vor dem Entfernen der organischen Anteile der Komponenten b) bzw. c) enthält:

a) Oxide oder in die entsprechenden Oxide überführbare Vorläuferverbindungen von Molybdän und Eisen, wobei das molare Verhältnis von Mo:Fe zwischen 1:1 und 5:1 liegt, sowie gegebenenfalls weitere metallische bzw. metalloxidische Komponenten oder in die entsprechenden Oxide überführbare Vorläuferverbindungen,

b) mindestens einen organischen Binder, vorzugsweise eine wässrige Dispersion von Copolymeren, insbesondere ausgewählt aus Vinylacetat/Vinyllaurat, Vinylacetat/Ethylen, Vinyacetat/Acrylat, Vinylacetat/Maleat, Styrol/Acrylat oder deren Gemischen,

c) und mindestens eine weitere Komponente ausgewählt aus der Gruppe bestehend aus $SiO_2$-Sol oder dessen Vorläufer, $Al_2O_3$-Sol oder dessen Vorläufer, $ZrO_2$-Sol oder dessen Vorläufer, $TiO_2$-Sol oder dessen Vorläufer, Wasserglas, MgO, Zement, monomeren, oligomeren oder polymeren Silanen, Alkoxysilanen, Aryloxysilanen, Acryloxysilanen, Aminosilanen, Siloxanen oder Silanolen.

So wurde überraschend gefunden, dass ein Beschichtungs- bzw. Schalenkatalysator, bei dem die Beschichtungszusammensetzung des Trägerkörpers neben der (katalytisch) aktiven Masse, enthaltend Oxide oder in die entsprechenden Oxide überführbare Vorläuferverbindungen von Molybdän und Eisen (Komponente a)), eine Kombination aus mindestens einem organischen Binder, der beim Tempern bzw. Calcinieren entfernt (ausgebrannt) wird, und mindestens einer weiteren Komponente, ausgewählt aus der Gruppe $SiO_2$-Sol oder dessen Vorläufer, $Al_2O_3$-Sol oder dessen Vorläufer $ZrO_2$-Sol oder dessen Vorläufer $TiO_2$-Sol oder dessen Vorläufer, Wasserglas, MgO, Zement, monomeres, oligomeres oder polymeres Silan, Alkoxysilan, Aryloxysilan, Acryloxysilan, Aminosilan, Siloxan oder Silanole, enthält, eine besonders hohe Abriebfestigkeit zeigt. Dabei wurde unerwartet gefunden, dass nicht nur die ungetemperten, d.h. die organischen Teile der Beschichtungszusammensetzung enthaltenden erfindungsgemäßen Beschichtungskatalysatoren besonders abriebfest sind, sondern nach dem Tempern bzw. Calcinieren der erfindungsgemäßen Katalysatoren weiterhin eine besonders hohe Abriebfestigkeit gegeben ist und eine für die Aktivität des Katalysators zur Oxidation von Methanol zu Formaldehyd besonders günstige Porosimetrie bereitgestellt wird. Dabei kann hierin der (noch nicht getemperte bzw. calcinierte) Katalysator, bei dem die organischen Anteile der Komponenten b) und c) noch nicht entfernt wurden, auch als Katalysator-Vorstufe bzw. Vorläufer-Katalysator angesehen werden. In dem fertigen Katalysator, wie er zur Oxidation von Methanol zu Formaldehyd eingesetzt wird, sind dann die organischen Anteile der Komponenten b) und c) entfernt, insbesondere durch Tempern bzw. Calcinieren.

Vereinfacht gesagt umfasst die Beschichtung in den erfindungsgemäßen Beschichtungskatalysatoren neben der aktiven Masse (Komponente a)) noch mindestens zwei als Haftungsvermittler wirkende Komponenten. Davon stellt mindestens eine Komponente einen organischen Binder dar (Komponente b)), der beim Tempern und Calcinieren des Katalysators entfernt werden kann. Die zweite Komponente (Komponente c)) weist zumindest einen anorganischen Anteil auf oder umfasst eine rein anorganische Verbindung, die nach dem Tempern bzw. Calcinieren des Beschichtungskatalysators Brücken zwischen den oxidischen Bestandteilen der aktiven Masse, d.h. den molybdän- bzw. eisenhaltigen Oxiden in vorteilhafter Weise bilden kann. Die anorganische Komponente bzw. deren anorganischer Anteil bildet, ohne dass die Erfindung auf die Richtigkeit des theoretischen Mechanismus beschränkt wäre, offenbar Verbindungen zwischen den Primärteilchen der Fe- bzw. Mo-Oxide, insbesondere des Eisenmolybdän-Mischoxids bzw. der anderen katalytisch aktiven Komponenten (a)). Die organische Komponente (b)) und, soweit vorhanden, die organischen Anteile der Komponente c) (siehe oben) ermöglichen dabei in enger Assoziation zu den Primärteilchen der katalytisch aktiven Komponente a), jedoch ohne deren Verbrückung durch die haftungsvermittelnde anorganischen Komponente bzw. deren anorganische Anteile zu beeinträchtigen, die Ausbildung von Poren und somit im fertigen Katalysator den optimalen Zutritt der Reaktanden zu den aktiven Zentren. In einer besonders bevorzugten Ausführung wird als Komponente b) ein organischer Binder aus der vorher genannten Gruppe eingesetzt. Die Verwendung dieses organischen Binders hat den Vorteil, dass sich der Beschichtungsvorgang in einer Fliessbettapparatur mit besonders geringen Sprühverlusten bewerkstelligen lässt. Zudem bildet sich ein vorteilhaftes Porengefüge nach dessen Entfernung aus.

Erfindungsgemäß umfasst der Beschichtungskatalysator zunächst einen inerten Trägerkörper. Hier können grundsätzlich alle dem Fachmann geläufigen inerten Trägerkörper verwendet werden. Vorzugsweise sollten die Trägerkörper jedoch im Wesentlichen unporös sein und eine BET-Oberfläche (bestimmt nach DIN 66131) von weniger als etwa 1 $m^2$/g aufweisen. Das Porenvolumen des inerten, im Wesentlichen unporösen Trägerkörpers soll vorzugsweise bei weniger als 0,1 ml/g, bestimmt gemäß DIN 66133, liegen. Beispiele für geeignete Trägerkörper sind beispielsweise Magnesiumsilicat (Steatit), Quarz ($SiO_2$), Porzellan, Magnesiumoxid, Zinndioxid, Siliciumcarbid, Rutil, Tonerde ($Al_2O_3$), Zirkoniumsilikat, Aluminiumsilikat, Cersilicat oder Mischungen dieser Trägermaterialien. Besonders bevorzugt sind Steatit-Trägerkörper. Es wurde gefunden, dass carbidhaltige oder gesinterte Eisenoxid-Trägerkörper schlechtere Ergebnisse liefern.

Nach einer bevorzugten erfindungsgemäßen Ausführungsform werden als inerte Trägerkörper Hohlzylinder eingesetzt. Nach einer weiter bevorzugten Ausführungsform weisen die verwendeten Trägerkörper-Hohlzylinder eine der folgenden Größen auf (Außendurchmesser x Höhe x Innendurchmesser):

```
6 x 5 x 3, 6 x 5 x 4, 6 x 4 x 4, 6 x 4 x 3, 5 x 5 x 3,
4 x 4 x 2, 4 x 4 x 1,5, 5 x 4 x 3, 5 x 5 x 2,5, 5 x 3 x 3, 4 x 3
x 1,5, 4 x 3 x 2, 3 x 3 x 1,5, 3 x 4 x 1,5 jeweils in mm.
```

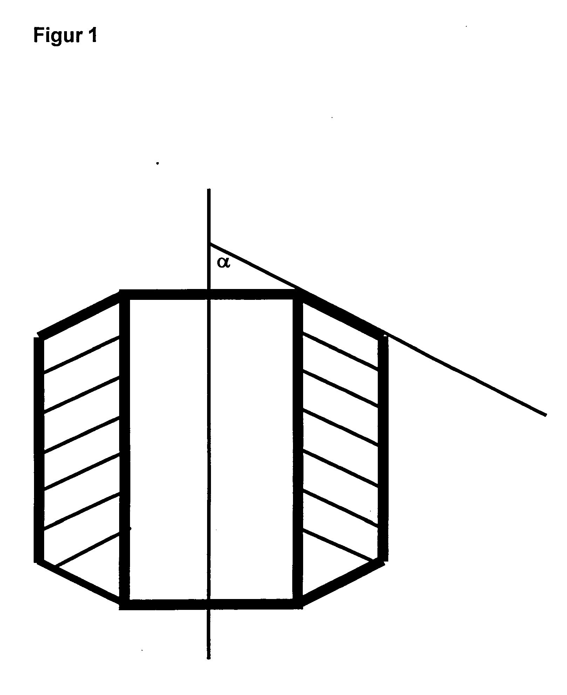

Weiterhin wurde überraschend gefunden, dass besonders gute Ergebnisse mit dem erfindungsgemäßen Katalysator bei der Oxidation von Methanol zu Formaldehyd erhalten werden können, wenn die Trägerkörper-Hohlzylinder mindestens einen, vorzugsweise genau einen zentralen Durchtrittskanal aufweisen und die Stirnseiten (Austrittsseiten des Durchtrittskanals) (nur) nach außen abgeflachte Kanten bzw. in einem spitzen Winkel (insbesondere 10-90°, weiter bevorzugt 30-85°, noch weiter bevorzugt 40-80°) zur Achse des Durchtrittskanals verlaufende Stirnflächen aufweisen, siehe Fig.1. Es können auch Formkörper gemäß der EP 1 127 618 A1 oder der WO 2005/037427 A1 verwendet werden.

Die vorstehend beschriebenen bevorzugten Trägerkörper in Form eines Zylinders mit Durchtrittskanal und stirnseitig nach außen abgeflachten Kanten bieten folgende Vorteile:

1. Die Formkörper bilden beim Beladen des Reaktors nicht so leicht lokal geordnete dichte Packungen, sondern sind eher unregelmäßig angeordnet, wobei im Gasstrom durch das Katalysatorbett mehr Turbulenzen entstehen, welche einer leicht einsetzenden Überhitzung insbesondere bei der Oxidation von Methanol zu Formaldehyd entgegenwirken. Die Verringerung der Überhitzung führt auch zu einer Verlängerung der Lebensdauer des Katalysators.

2. Bei der Herstellung des Katalysators im bevorzugten Fliessbettcoater besteht weniger Bruch an den Kanten als bei Verwendung von zylindrischen Körpern ohne abgeflachte Kanten.

3. Beim Füllen des Katalysators verkanten die Träger mit abgeflachten Kanten weniger als bei Verwendung von zylindrischen Körpern ohne abgeflachte Kanten. Dies gilt insbesondere für den Fall, dass der Wert von Reaktionsdurchmesser zu Katalysatordurchmesser zwischen etwa 2 und 5 liegt. Das Verkanten der Partikel führt zu sogenannten Fülllücken.

Auf den inerten Trägerkörper ist mindestens eine Schicht (Schale) einer Beschichtungszusammensetzung aufgebracht, die zunächst als aktive Komponente a) mindestens eine Molybdänverbindung und mindestens eine Eisenverbindung oder mindestens eine Fe- und Mohaltige Verbindung enthält, wobei das molare Verhältnis von Mo:Fe vorzugsweise zwischen etwa 1:1 und 5:1 liegt. Bevorzugt werden Oxide von Molybdän bzw. Eisen und/oder deren Mischoxide (wie $Fe_2(MoO_4)_3$) oder Verbindungen, die in die entsprechenden Oxide bzw. Mischoxide überführt werden können, wie beispielsweise Acetate, Oxalate, Acetylacetonate, Citrate, Nitrate, Chloride, Phosphate, Sulfate oder Ammoniumverbindungen des Eisens bzw. Molybdäns, eingesetzt. Nach einer besonders bevorzugten erfindungsgemäßen Ausführungsform wird ein Eisenmolybdän-Mischoxid ($Fe_2(MoO_4)_3$) als eisenhaltige Verbindung und $MoO_3$ als molybdänhaltige Verbindung verwendet. Daneben können in der vorstehenden Komponente a) auch gegebenenfalls weitere metallische bzw. metalloxidische Komponenten oder Verbindungen ,die in die entsprechenden Oxide bzw. Mischoxide überführt werden können, enthalten sein, die dem Fachmann im Hinblick auf die Verwendung des Katalysators zur Oxidation von Methanol zu Formaldehyd geläufig sind. Nicht beschränkende Beispiele sind die Lanthanidenmetalle bzw. deren Oxide. Bevorzugt sind Titan, Antimon, Zinn, Nickel, Cer, Aluminium, Calcium, Magnesium, Chrom, Vanadium, Niob, Silber und/oder Mangan, die Fe und Mo auch teilweise ersetzen können.

Erfindungsgemäß umfasst die Beschichtung des erfindungsgemäßen Katalysators (vor dem Entfernen der organischen Anteile) mindestens einen organischen Binder (Komponente b)). Dieser Binder verbessert die Abriebfestigkeit der Beschichtung und trägt zu der guten Haftung der Beschichtung an dem inerten Trägerkörper bei. Zudem wird bei Verwendung eines organischen Binders wie hierin definiert auch ein besonders geringer Sprühverlust und somit eine besonders hohe Aufbringausbeute bei der Beschichtung der inerten Trägerkörper im Fliessbettverfahren (Fliessbettcoater) erzielt. Zusätzlich trägt er zur Ausbildung vorteilhafter Poren nach dem Entfernen der organischen Anteile der Beschichtungszusammensetzung bei. Es wurde nun gefunden, dass besonders vorteilhafte Ergebnisse mit wässrigen Dispersionen von Copolymeren, insbesondere Vinylacetat/Vinyllaurat, Vinylacetat/Ethylen, Vinylacetat/Acrylat, Vinylacetat/Maleat, oder Styrol/Acrylat als organische Binder erhalten werden. Solche Copolymere sind dem Fachmann als solche geläufig, können nach Standardverfahren hergestellt werden und sind kommerziell erhältlich. Geeignete Dispersionen weisen vorzugsweise einen Feststoffgehalt im Bereich von 20 bis 80 Gew.-%, bevorzugt 40 - 60 Gew.-% auf. Geeignete Bindemittel umfassen z.B. Bindemitteldispersionen worin das Bindemittel ein Copolymer eines alpha-Olefins und eines Vinyl-$C_2$-$C_4$-Carboxylats ist, dessen Vinyl-$C_2$-$C_4$-Carboxylatgehalt wenigstens 62 Mol-% beträgt wie beispielsweise in der WO2005/011862 genannt.

Daneben enthält die Beschichtungszusammensetzung der erfindungsgemäßen Beschichtungskatalysatoren mindestens eine weitere Komponente (c)) ausgewählt aus der Gruppe bestehend aus $SiO_2$-Sol oder dessen Vorläufer, $Al_2O_3$-Sol oder dessen Vorläufer $ZrO_2$-Sol oder dessen Vorläufer $TiO_2$-Sol oder dessen Vorläufer, Wasserglas, MgO, Zement, monomeres, oligomeres oder polymeres Silan, Alkoxysilan, Aryloxysilan, Acryloxysilan, Aminosilan, Siloxan oder Silanole. Diese Verbindungen sind dem Fachmann als solche geläufig, können nach Standardverfahren hergestellt werden und sind kommerziell erhältlich.

Unter den besonders bevorzugten Komponenten sind hier die anorganischen Sole, insbesondere $TiO_2$-Sole, Ceroxid-Sole und $ZrO_2$-Sole und zu nennen, da hier besonders gute Abriebfestigkeiten und zugleich hohe Aktivitäten der fertigen Katalysatoren bei der Oxidation von Methanol zu Formaldehyd erhalten wurden.

Je nach Sol liegt der Feststoffgehalt zwischen 10 und 50 Gew.-%, beispielsweise sind $SiO_2$-Sole mit 20 - 40 Gew.-%, $ZrO_2$-Sole mit 20 Gew.-%, $CeO_2$-Sole mit 15 -25 Gew.-% Feststoffgehalt und $TiO_2$-Sole mit 10 - 20 Gew.-% Feststoffgehalt bevorzugt. $TiO_2$-Sole sind besonders bevorzugt.

Es wurde unerwartet gefunden, dass die vorstehenden Komponenten b) und c) bei der Erzeugung einer optimalen Abriebfestigkeit und Porosimetrie des Katalysators zusammenwirken. Durch die erfindungsgemäß eingesetzte Haftvermittlerkombination (Kombination aus der mindestens einen Komponente b) und mindestens einer Komponente c)) wird sowohl ein Haften und vorteilhaftes Aufbringen der Beschichtungszusammensetzung bzw. der aktiven Masse (Komponente a)) auf dem Formkörper, insbesondere im bevorzugten Fliessbettverfahren, als auch eine offenporige Struktur und gute Haftung der aktiven Masse nach dem Tempern bzw. Calcinieren des Katalysators bereitgestellt.

Nach einer bevorzugten erfindungsgemäßen Ausführungsform liegt das integrale Porenvolumen (bestimmt mit Hg-Porosimetrie, DIN 66133) zwischen etwa 100 - 800 $mm^3$/g, bevorzugt zwischen etwa 200 und 700 $mm^3$/g besonders bevorzugt zwischen etwa 250 und 600 $mm^3$ / g. Der nach dieser Methode ermittelte mittlere Porenradius liegt vorzugsweise zwischen etwa 50 und 1000 nm, bevorzugt zwischen etwa 100 und 700 nm, besonders bevorzugt zwischen etwa 150 und 500 nm.

Weiterhin hat sich überraschend herausgestellt, dass der erfindungsgemäße ungetemperte Katalysator (Vorläufer-Katalysator), d.h. vor dem Entfernen der organischen Anteile der Beschichtungszusammensetzung eine stark verbesserte Transport- und Füllstabilität aufweist. Ein Aspekt der vorliegenden Erfindung betrifft somit auch die Verwendung eines erfindungsgemäßen Katalysators vor dem Entfernen der organischen Anteile der Beschichtungszusammensetzung (d.h. als Vorläufer-Katalysator) für den Transport von dem Produktionsort des Katalysators (bzw. Vorläufer-Katalysators) zum Einsatzort des Katalysators, insbesondere bei der Oxidation von Methanol zu Formaldehyd. Ein weiterer Aspekt betrifft die Verwendung eines solchen erfindungsgemäßen Katalysators vor dem Entfernen der organischen Anteile der Beschichtungszusammensetzung (d.h. als Vorläufer-Katalysator) zum Befüllen eines Reaktors, insbesondere zur Oxidation von Methanol zu Formaldehyd.

Nach einer bevorzugten erfindungsgemäßen Ausführungsform wird als Komponente c) mindestens ein Sol mit einer Partikelgröße von 1 bis 100 nm, vorzugsweise 2 bis 50 nm, besonders bevorzugt < 40 nm eingesetzt. Die Bestimmung dieser Partikelgrößen erfolgte nach ASTM B822-97. Alternativ kann auch ISO 13320-1 verwendet werden.

Besonders bevorzugt weist die Komponente c) eine kleinere mittlere Teilchengröße auf als die Komponente (n) a).

Besonders bevorzugt wird hierbei, dass Komponente c) ein $ZrO_2$-Sol, ein $CeO_2$-Sol, ein $TiO_2$-Sol oder eine Mischung aus zwei oder aller drei der vorstehenden Komponenten umfasst. Weiterhin wurde überraschend gefunden, dass besonders gute Abriebfestigkeiten mit einem $TiO_2$-Sol erhalten wurde, das mit Salpetersäure oder mit Zitronensäure stabilisiert worden war. Des Weiteren wurden gute Abriebfestigkeiten mit einem $CeO_2$-Sol, das mit Essigsäure stabilisiert ist bzw. einer Mischung aus dem $TiO2$-Sol und dem $CeO_2$-Sol gefunden. Besonders vorteilhaft ist dabei, wenn das $TiO_2$-Sol im Überschuss gegenüber dem $CeO_2$-Sol vorliegt. Des Weiteren wurden gute Abriebfestigkeiten mit einem $ZrO_2$-Sol gefunden, das mit Acetat stabilisiert worden war.

Nach einer bevorzugten erfindungsgemäßen Ausführungsform liegt der Feststoffanteil der Komponente c) (also z.B. $ZrO_2$ oder $TiO_2$) nach der Entfernung der organischen Anteile der Komponenten b) bzw. c), insbesondere nach dem Calcinieren bzw. Tempern des Katalysators (d.h. im fertigen Katalysator), zwischen etwa 0,01 und 30 Gew.-%, bevorzugt zwischen 0,05 und 20 Gew.-%, weiter bevorzugt zwischen 0,1 und 10 Gew.-%. Die entsprechenden Einsatzmengen an Komponente c) können somit vom Fachmann leicht je nach der verwendeten Komponente c) und Komponente a) ermittelt werden.

Weiterhin wird erfindungsgemäß bevorzugt, dass der Feststoffanteil der Komponente b), zwischen 5 und 30 Gew.-%, bevorzugt zwischen etwa 8 und 25 Gew.-% liegt, wobei sich diese Angaben natürlich auf den Zustand vor dem Entfernen der organischen Anteile der Beschichtungszusammensetzung beziehen.

Nach einer weiteren bevorzugten Ausführungsform der Erfindung liegt der Aktivmassenanteil zwischen etwa 3 und 60 Gew.-%, bevorzugt zwischen etwa 3 und 50 Gew.-%, weiter bevorzugt zwischen etwa 5 und 40 Gew.-% Bei den üblicherweise verwendeten Geometrien der inerten Trägerkörper ergibt sich dadurch eine erfindungsgemäß bevorzugte Dicke der Beschichtung auf dem inerten Trägerkörper zwischen etwa 30 und 1000 $\mu$m, bevorzugt zwischen etwa 100 und 700 $\mu$m.

Nach einer bevorzugten erfindungsgemäßen Ausführungsform liegt bei dem erfindungsgemäßen Katalysator nach dem Entfernen der organischen Anteile der Beschichtungszusammensetzung das auf die Aktivmasse bezogene integrale Porenvolumen (nach DIN 66133) bei mehr als etwa 0,15 ml/$g_{Aktivmasse}$, bevorzugt bei mehr als 0,2 ml/$g_{Aktivmasse}$.

Ein weiterer Aspekt der vorliegenden Erfindung betrifft ein Verfahren zur Herstellung eines erfindungsgemäßen Beschichtungskatalysators, insbesondere zur Oxidation von Methanol zu Formaldehyd, umfassend die folgenden Schritte:

a) Bereitstellen eines inerten, vorzugsweise im wesentlichen unporösen Trägerkörpers,

b) Herstellen einer wässrigen Suspension, enthaltend die Komponenten a), b), c) wie in den vorstehenden Ansprüchen definiert,

c) Aufbringung der wässrigen Suspension gemäß Schritt b) auf den inerten Trägerkörper, vorzugsweise im Fließbettverfahren.

In einem ersten Schritt wird ein inerter, vorzugsweise im Wesentlichen unporöser Trägerkörper wie vorstehend beschrieben, bereitgestellt.

Weiterhin wird eine wässrige Suspension, enthaltend die Komponenten a), b), c), wie vorstehend beschrieben, bereitgestellt. Hierbei hat es sich erfindungsgemäß als besonders vorteilhaft herausgestellt, dass alle Komponenten a), b) und c) in Teilchenform, entweder suspendiert oder dispergiert und nicht in gelöster Form vorliegen. Der Feststoffgehalt in der Suspension / Dispersion liegt bevorzugt zwischen 5 und 40 Gew.-%, besonders bevorzugt zwischen 10 und 30 Gew.-%.

Bei dem erfindungsgemäßen Verfahren wird es bevorzugt, dass die bereitgestellte wässrige Suspension mittels eines Fliessbettverfahrens auf den inerten Trägerkörper aufgebracht wird. Dabei wird die Verwendung eines Fliessbettcoaters bevorzugt, wie er beispielsweise in der DE-A-12 80 756, der DE-A-198 28 583, der DE-A-197 09 589, der DE 40 06 935 A1, der DE 103 44 845 A1 oder der WO 2005/030388 beschrieben ist. Es hat sich erfindungsgemäß gezeigt, dass die wässrige Suspension mit der Beschichtungszusammensetzung mittels eines Fliessbettverfahrens, wie vorstehend ausgeführt, besonders gleichmäßig und gut haftend sowie mit unerwartet geringen Sprühverlusten auf den unporösen Trägerkörper aufgebracht werden kann.

Weiterhin hat es sich als vorteilhaft herausgestellt, dass der pH-Wert der Suspension dem Stabilitätsbereich der Komponente b) und Komponente c) angepasst wird. Es hat sich gezeigt, dass hierzu allgemein ein pH-Wert der auf die Trägerkörper aufzubringenden Suspension zwischen etwa 1 und 5 von Vorteil ist. Es hat sich auch gezeigt, dass ein pH-Wert von etwa 3 - 5 im Falle der Verwendung eines acetatstabilisierten $ZrO_2$-Sols bzw. ein pH-Wert zwischen 1 und 5 vorzugsweise zwischen 1 und 3 im Falle der Verwendung eines mit Salpetersäure stabilisierten $TiO_2$-Sols von Vorteil ist. Im Falle des mit Essigsäure stabilisierten $CeO_2$-Sols ist ein pH Wert von 2 - 4 besonders vorteilhaft.

Weiterhin hat sich gezeigt, dass besonders günstige Ergebnisse erhalten werden, wenn die Komponente a) vor dem Auftragen auf den inerten Trägerkörper nicht auf über 200°C erhitzt wird, insbesondere nicht getempert bzw. calciniert wird.

Nach einer weiteren bevorzugten erfindungsgemäßen Ausführungsform erfolgt die Aufbringung der wässrigen Suspension auf die inerten Trägerkörper im Fliessbettverfahren bei einer Temperatur von weniger als 100°C, insbesondere bei weniger als 80°C, weiter bevorzugt weniger als 70°C.

Bevorzugt weisen die Teilchen der Komponenten a), b) und c) in der wässrigen Suspension einen $D_{90}$-Wert der Teilchengröße von weniger als 50 $\mu$m, bevorzugt weniger als 30 $\mu$m auf. Die Einhaltung dieser Teilchengröße trägt zu einer besonders gleichmäßigen und abriebfesten Beschichtung auf den inerten Trägerkörpern bei und führt zu geringen Sprühverlusten während des Beschichtungsvorgangs. Sofern nicht ohnehin für die Komponenten a), b) und c) Materialien eingesetzt werden, die die vorstehende Teilchengröße aufweisen, kann diese durch herkömmliches Mahlen bzw. Zerkleinern vor, während oder nach der Herstellung der wässrigen Suspension eingestellt werden.

Nach einer weiteren bevorzugten Ausführungsform wird nach dem Aufbringen der Beschichtungszusammensetzung auf den inerten Trägerkörper der beschichtete Trägerkörper thermisch behandelt. Allgemein kann jede dem Fachmann geläufige Temperatur und Zeitdauer zur Calcinierung bzw. zum Tempern verwendet werden. In vielen Fallen wird eine Temperatur zwischen 200 und 600 °C, insbesondere 200 und 550 °C vorteilhaft sein. Die Dauer der Calcinierung bzw. des Temperns wird vorzugsweise zwischen 0,5 und 20 h, insbesondere zwischen 1 und 15 h liegen. Nach einer bevorzugten Ausführungsform wird der beschichtete Trägerkörper in einen geeigneten Wärmeschrank gegeben, z.B. einen Hordenofen mit Blechen, die den beschichteten Trägerkörper als Partikelschüttung mit einer bevorzugten Schüttungshöhe von 1 - 5 cm, besonders bevorzugt von 1 - 3 cm enthält. Der Wärmeschrank wird dann mit konstanter Rate in einem Zeitraum von 1 h bis 20 h, bevorzugt 5 h bis 15 h von Raumtemperatur auf eine Temperatur T1 zwischen 200 °C bis 300 °C erhitzt. Die Temperatur T1 wird dann für die Dauer von 1 h bis 5 h konstant gehalten. Anschließend wird die Temperatur mit konstanter Rate in einem Zeitraum von 1 h 10 h bevorzugt von 1 h bis 5 h ausgehend von der Temperatur T1 auf eine Endtemperatur T2 zwischen 300 bis 600 °C, bevorzugt 350 bis 550 °C, besonders bevorzugt 400 °C bis 550 °C erhitzt. Die Temperatur T2 wird für eine Zeitdauer von 1 - 10 h, bevorzugt von 2 - 5 h gehalten und der Wärmeschrank dann abgekühlt. Während der thermischen Behandlung kann es vorteilhaft sein, den Gasraum des Wärmeschranks mit Luft oder einer Mischung zeitlich unterschiedlicher Zusammensetzung aus Luft, Stickstoff und gegebenenfalls Wasserdampf zu durchströmen.

Die gemäß der vorstehenden Beschreibung als Komponente b) bevorzugten organischen Binder weisen den Vorteil auf, dass sie bereits bei den vorstehenden relativ niedrigen Beschichtungstemperaturen im Fliessbettcoater sehr gut zur Haftung und Abriebfestigkeit der Beschichtungszusammensetzung beitragen, und gleichzeitig bei den zur Erhitzung bzw. Calcinierung des Beschichtungskatalysators bevorzugten Temperaturen nach dem Aufbringen der Beschichtung auf den inerten Trägerkörper entfernt werden können, ohne die Haftung und Abriebfestigkeit der Beschichtung zu beeinträchtigen, um zu einem vorteilhaften Porengefüge beizutragen.

Nach einer bevorzugten Erfindung erfolgt das vorstehende Erhitzen nach dem Aufbringen der Beschichtung auf den inerten Trägerkörper in einer Atmosphäre aus Luft, Inertgas und/oder Wasserdampf. Soweit das Erhitzen in Anwesenheit von Wasserdampf erfolgt, wird ein Wasserdampfgehalt von mehr als etwa 10 Vol.-%, bevorzugt zwischen etwa 20 und 60 Vol.-% der verwendeten Atmosphäre bevorzugt.

Soweit, wie vorstehend ausgeführt, nach einem bevorzugten Aspekt der vorliegenden Erfindung der erfindungsgemäße Beschichtungskatalysator vor dem Entfernen der organischen Anteile der Beschichtungszusammensetzung zum Befüllen des Reaktors selbst verwendet wird, wurde im Rahmen der vorliegenden Erfindung auch gefunden, dass die Entfernung der organischen Anteile der Beschichtungszusammensetzung (mittels Erhitzen bzw. Tempern oder Calcinieren des Katalysators) in vorteilhafter Weise auch direkt im Reaktor selbst, insbesondere in einem $O_2$- und $N_2$-haltigen Gasstrom und/oder unter den für den fertigen Katalysator vorgesehenen Betriebsbedingungen wie den Bedingungen der vorgesehenen Methanoloxidation zu Formaldehyd stattfinden kann.

Ein weiterer erfindungsgemäßer Aspekt betrifft einen Katalysator, erhältlich nach dem vorstehenden Verfahren. Derartige Katalysatoren zeichnen sich gegenüber Katalysatoren des Standes der Technik durch eine erhöhte Abriebfestigkeit aus. Weiterhin ist durch eine vorteilhafte Porosimetrie nach dem Entfernen der organischen Anteile der Beschichtungszusammensetzung für die Katalysatoren eine hohe Aktivität und Formaldehydselektivität insbesondere bei der Oxidation von Methanol gewährleistet.

Somit betrifft ein weiterer erfindungsgemäßer Aspekt der vorliegenden Erfindung die Verwendung des vorstehend beschriebenen Beschichtungskatalysators zur Oxidation von Methanol zu Formaldehyd, insbesondere in einem Festbettverfahren. Ein geeignetes Verfahren ist beispielsweise aus der DE 103 61 517, der EP 0 001 570 und der US 3,852,361 bekannt. Es können auch andere dem Fachmann geläufige Verfahren zur Herstellung von Formaldehyd verwendet werden. Jedoch sind grundsätzlich auch andere Anwendungen des Katalysators, insbesondere bei partiellen (Gasphasen)-Oxidationen von Kohlenwasserstoffen nicht ausgeschlossen.

Ein weiterer erfindungsgemäßer Aspekt betrifft die Verwendung einer Kombination der vorstehend beschriebenen Komponenten b) und c) zur Erhöhung der Abriebfestigkeit eines Beschichtungskatalysators, insbesondere eines Katalysators zur Oxidation von Methanol zu Formaldehyd.

Figur 1 zeigt einen Querschnitt durch einen erfindungsgemäß bevorzugt verwendeten Trägerkörper mit stirnseitig nach außen abgeflachten Kanten. Der spitze Winkel $\alpha$ zwischen der Achse der zentralen Durchtrittsöffnung und den Stirnflächen liegt jeweils bei etwa 60 Grad.

Begriffsdefinitionen:

a) **Aktivmassenanteil** = Masse Aktivmasse (Komponente a)) / (Masse Aktivmasse (Komponente a)) + Masse Trägerkörper)

b) **Feststoffanteil Komponente b** = Masse Feststoff im org. Bindemittel (Komponente b)) / (Masse Feststoff im org. Bindemittel (Komponente b)) + Masse Aktivmasse (Komponente a)))

c) **Feststoffanteil Komponente c =** Masse Feststoff in Komponente c) / (Masse Feststoff in Komponente c )+ Masse Aktivmasse (Komponente a)))

Erfindungsgemäß wurden folgende Bestimmungsmethoden verwendet:

1. Test zur Bestimmung der Haftfähigkeit der aktiven Masse (Abriebstest):

Die Haftfähigkeit der aktiven Masse wurde in einem Gerät der Fa. ERWEKA, Typ TAR 10 untersucht. Dazu wurde bei einer Umdrehungszahl von 10 und einer Umdrehungsgeschwindigkeit von 75 / min eine Menge von 50 g gecoatetem Katalysator rotiert. Es wurde danach der Abrieb als Verhältnis der losen, nicht mehr auf dem Trägerkörper gebunden Beschichtung zur insgesamt aufgetragenen Beschichtung bestimmt.

2. BET-Oberfläche:

Die Bestimmung erfolgt nach der BET-Methode gemäß DIN 66131; eine Veröffentlichung der BET-Methode findet sich auch in J. Am. Chem. Soc. 60, 309 (1938).

3. Porenradienverteilung:

Die Bestimmung der Porenradienverteilung erfolgte Quecksilberporosimetrie gemäß DIN 66133; maximaler Druck: 2.000 bar, Porosimeter 4000 (Firma Porotec, DE), nach Angaben des Herstellers.

4. Bestimmung der Teilchengrößen (Partikelgrößen):

Die Bestimmung der Partikelgrößen erfolgte nach der Laserbeugungsmethode mit einem Fritsch Particle Sizer Analysette 22 Economy (Fa. Fritsch, DE) nach den Angaben des Herstellers, auch bezüglich der Probenvorbehandlung: die Probe wird in deionisiertem Wasser ohne Zusatz von Hilfsmitteln homogenisiert und 5 Minuten mit Ultraschall behandelt. Die angegebenen D-Werte sind auf das Probenvolumen bezogen.

Die Erfindung wird nun anhand der nachstehenden nicht beschränkenden Beispiele näher erläutert:

Beispiel 1 (erfindungsgemäß) :

Zur Herstellung des erfindungsgemäßen Katalysators mit einem Aktivmassenanteil (Komponente a)) von 20 Gew.-%, einem Anteil eines organischen Bindemittels (Komponente b)) von 20 Gew.-% und einem Anteil der anorganischen haftungsvermittelnden Komponente (c)) von 1 Gew.-% wurden in einem sogenannten Wirbelbettcoater 700 g Steatitkörper in Form von Hohlzylindern der Abmessung 5 x 5 x 2,5 mm mit einer Suspension, die wie folgt hergestellt wurde, beschichtet:
In einem Glasbehälter werden 1100 ml VE-Wasser vorgelegt. 184 g des Aktivmassenpulvers (Mischung aus $Fe_2(MoO_4)_3$ und $MoO_3$) werden unter Rühren suspendiert. Zur besseren Homogenisierung wird die Suspension 3 min auf Stufe 6 mit dem Ultra - Turrax behandelt. Unter Rühren werden 9,22 g des $ZrO_2$-Sols (20 % Feststoffgehalt, acetatstabilisiert, Fa. Nyacol, Tradename: NYACOL Zirconia (Acetate)) dazugegeben. Der pH-Wert der Suspension wird mit einer 25 %igen Ammoniaklösung auf 4 eingestellt. Zu der Suspension werden 92 g des organischen Bindemittels (50 %ige Dispersion von Wasser und Vinylacetat / Ethylencopolymer, Vinnapas EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fliessbett aufgebracht.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 1,3 Gew.-%, der Abrieb des bei 500 °C calcinierten Katalysators betrug 3,1 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,42 ml/g, für den bei 500 °C calcinierten Katalysator 0,37 ml / g.

Beispiel 2 (erfindungsgemäß):

Zur Herstellung des erfindungsgemäßen Katalysators mit einem Aktivmassenanteil (Komponente a)) von 20 Gew.-%, einem Anteil eines organischen Bindemittels (Komponente b)) von 20 Gew.-% und einem Anteil der anorganischen haftungsvermittelnden Komponente (c)) von 4,7 Gew.-% wurden in einem sogenannten Wirbelbettcoater 700 g Steatitkörper in Form von Hohlzylindern der Abmessung 5 x 5 x 2,5 mm mit einer Suspension, die wie folgt hergestellt wurde, beschichtet:
In einem Glasbehälter werden 1100 ml VE-Wasser vorgelegt. 184 g des Aktivmassenpulvers (Mischung aus $Fe_2(MoO_4)_3$ und $MoO_3$) werden unter Rühren suspendiert. Zur besseren Homogenisierung wird die Suspension 3 min auf Stufe 6 mit dem Ultra - Turrax behandelt. Unter Rühren werden 76 g des $TiO_2$-Sols (12 % Feststoffgehalt, Salpetersäurestabilisiert, Fa. Sachtleben, Tradename: Hombikat XXS 100) dazugegeben. Der pH-Wert der Suspension betrug 2,2. Zu der Suspension werden 92 g des organischen Bindemittels (50 %ige Dispersion von Wasser und Vinylacetat / Ethylencopolymer, Vinnapas EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fliessbett aufgebracht.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 2,7 Gew.-%, der Abrieb des bei 500 °C calcinierten Katalysators betrug 5,3 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,44 ml/g, für den bei 500 °C calcinierten Katalysator 0,38 ml / g.

Beispiel 3 (erfindungsgemäß) :

Zur Herstellung des erfindungsgemäßen Katalysators mit einem Aktivmassenanteil (Komponente a)) von 26 Gew.-%, einem Anteil eines organischen Bindemittels (Komponente b)) von 20 Gew.-% und einem Anteil der anorganischen haftungsvermittelnden Komponente (c)) von 4,7 Gew.-% wurden in einem sogenannten Wirbelbettcoater 700 g Steatitkörper in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 245 g des Aktivmassenpulvers (Mischung aus $Fe_2(MoO_4)_3$ und $MoO_3$) werden unter Rühren suspendiert. Zur besseren Homogenisierung wird die Suspension 3 min auf Stufe 6 mit dem Ultra - Turrax behandelt. Unter Rühren werden 46 g des Ceroxid-Sols (20 % Feststoffgehalt, 3 % Essigsäure) dazugegeben. Der pH-Wert der Suspension wird mit einer 25 %igen Ammoniaklösung auf 4 eingestellt. Zu der Suspension werden 122 g des organischen Bindemittels (50 %ige Dispersion von Wasser und Vinylacetat / Ethylencopolymer, Vinnapas EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fliessbett aufgebracht.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 0,7 Gew.-%, der Abrieb des bei 500 °C calcinierten Katalysators betrug 1,3 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,41 ml/g, für den bei 500 °C calcinierten Katalysator 0,37 ml / g.

Beispiel 4 (erfindungsgemäß) :

Zur Herstellung des erfindungsgemäßen Katalysators mit einem Aktivmassenanteil (Komponente a)) von 20 Gew.-%, einem Anteil eines organischen Bindemittels (Komponente b)) von 20 Gew.-% und einem Anteil der anorganischen haftungsvermittelnden Komponente (c)) von 4,7 Gew.-% wurden in einem sogenannten Wirbelbettcoater 700 g Steatitkörper in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 184 g des Aktivmassenpulvers (Mischung aus $Fe_2(MoO_4)_3$ und $MoO_3$) werden unter Rühren suspendiert. Zur besseren Homogenisierung wird die Suspension 3 min auf Stufe 6 mit dem Ultra - Turrax behandelt. Unter Rühren werden 46 g des SiO2-Sols (GRACE Davidson, 30% Feststoffgehalt, Tradename: Ludox AS-30) dazugegeben. Der pH-Wert der Suspension wird mit einer 25 %igen Ammoniaklösung auf 4 eingestellt. Zu der Suspension werden 92 g des organischen Bindemittels (50 %ige Dispersion von Wasser und Vinylacetat / Ethylencopolymer, Vinnapas EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fliessbett aufgebracht.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 2,7 Gew.-%, der Abrieb des bei 500 °C calcinierten Katalysators betrug 4,2 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,39 ml/g, für den bei 500 °C calcinierten Katalysator 0,35 ml / g.

Beispiel 5 (Vergleich):

Zur Herstellung des Vergleichskatalysators mit einem Aktivmassenanteil (Komponente a)) von 21 Gew.-%, und einem Anteil des organischen Bindemittels (Komponente b)) von 20 Gew.-% wurden in einem sogenannten Wirbelbettcoater 700 Steatitkörper in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 184 g des Aktivmassenpulvers (Mischung aus $Fe_2(MoO_4)_3$ und $MoO_3$) werden unter Rühren suspendiert. Zur besseren Homogenisierung wird die Suspension 3 min auf Stufe 6 mit dem Ultra - Turrax behandelt. Der pH-Wert der Suspension wird mit einer 25 %igen Ammoniaklösung auf 4 eingestellt. Zu der Suspension werden 92 g des organischen Bindemittels (50 %ige Dispersion von Wasser und Vinylacetat / Ethylencopolymer, Vinnapas EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C im Fliessbett auf die Steatitkörper aufgebracht.

Bei einer Temperatur von 400 °C bzw. 500 °C wurde für eine Zeitdauer von 2 Stunden ein Teil des gecoateten Katalysators thermisch behandelt und dabei das organische Bindemittel entfernt. Der Abrieb des bei 400 °C calcinierten Katalysators betrug 37,9 Gew.-%, der Abrieb des bei 500 °C calcinierten Katalysators betrug 38,3 Gew.-%. Der Abrieb des uncalcinierten Katalysators lag etwas unter 1 % und deutlich höher als bei den vorstehenden erfindungsgemäßen Katalysatoren.

Das mittels Hg-Porosimetrie gemessene integrale Porenvolumen beträgt für den bei 400 °C calcinierten Katalysator 0,46 ml/g, für den bei 500 °C calcinierten Katalysator 0,42 ml / g.

**Patentansprüche**

1. Beschichtungskatalysator, insbesondere zur Oxidation von Methanol zu Formaldehyd, der auf einem inerten, vorzugsweise im wesentlichen unporösen Trägerkörper mindestens eine Beschichtung aufweist, die vor dem Entfernen der organischen Anteile der Komponenten b) bzw. c) enthält:

   a) Oxide oder in die entsprechenden Oxide überführbare Vorläuferverbindungen von Molybdän und Eisen, wobei das molare Verhältnis von Mo:Fe zwischen 1:1 und 5:1 liegt, sowie gegebenenfalls weitere metallische bzw. metalloxidische Komponenten oder in die entsprechenden Oxide überführbare Vorläuferverbindungen,
   b) mindestens einen organischen Binder, vorzugsweise eine wässrige Dispersion von Copolymeren, insbesondere ausgewählt aus Vinylacetat/Vinyllaurat, Vinylacetat/Ethylen, Vinyacetat/Acrylat, Vinylacetat/Maleat, Styrol/Acrylat oder deren Gemischen,
   c) und mindestens eine weitere Komponente ausgewählt aus der Gruppe bestehend aus $SiO_2$-Sol oder dessen Vorläufer, $Al_2O_3$-Sol oder dessen Vorläufer, $ZrO_2$-Sol oder dessen Vorläufer, $TiO_2$-Sol oder dessen Vorläufer, Wasserglas, MgO, Zement, monomeren, oligomeren oder polymeren Silanen, Alkoxysilanen, Aryloxysilanen, Acryloxysilanen, Aminosilanen, Siloxanen oder Silanolen.

2. Beschichtungskatalysator gemäß Anspruch 1, wobei es sich bei dem organischen Binder um eine wässrige Dispersion von Copolymeren, insbesondere ausgewählt aus Vinylacetat/Vinyllaurat, Vinylacetat/Ethylen, Vinylacetat/Acrylat, Vinylacetat/Maleat, Styrol/Acrylat oder deren Gemischen handelt.

3. Beschichtungskatalysator nach einem der vorstehenden Ansprüche, **dadurch gekennzeichnet, dass** Molybdän und Eisen als Oxide, Mischoxide oder eine Mischung aus Mischoxid und den reinen Oxiden vorliegen.

4. Beschichtungskatalysator nach einem der vorstehenden Ansprüche, **dadurch gekennzeichnet, dass** als Komponente c) mindestens ein Sol mit einer Partikelgröße von 1 bis 100 nm, vorzugsweise 2 bis 50 nm, weiter bevorzugt 3 - 40 nm eingesetzt wird.

5. Beschichtungskatalysator nach einem der vorstehenden Ansprüche, **dadurch gekennzeichnet, dass** die Komponente c) $TiO_2$-Sol oder $ZrO_2$-Sol oder $CeO_2$-Sol oder eine Mischung von zwei oder aller drei genannten Komponenten umfasst.

6. Beschichtungskatalysator nach einem der vorstehenden Ansprüche, **dadurch gekennzeichnet, dass** das $ZrO_2$-Sol acetatstabilisiert ist.

7. Beschichtungskatalysator nach einem der vorstehenden Ansprüche, **dadurch gekennzeichnet, dass** das $TiO_2$-Sol entweder mit Salpetersäure oder Zitronensäure stabilisiert ist.

8. Beschichtungskatalysator nach einem der vorstehenden Ansprüche, **dadurch gekennzeichnet, dass** das $CeO_2$-Sol mit Essigsäure stabilisiert ist.

9. Beschichtungskatalysator nach einem der vorstehenden Ansprüche, **dadurch gekennzeichnet, dass** der Feststoffanteil der Komponente c), zwischen 0,01 und 30 Gew.-%, bevorzugt zwischen 0,05 und 20 Gew.-%, weiter bevorzugt zwischen 0,1 und 10 Gew.-% liegt.

10. Beschichtungskatalysator nach einem der vorstehenden Ansprüche, **dadurch gekennzeichnet, dass** der Feststoffanteil der Komponente b) zwischen 5 und 30 Gew.-%, bevorzugt zwischen 8 und 25 Gew.-% liegt.

11. Beschichtungskatalysator nach einem der vorstehenden Ansprüche, **dadurch gekennzeichnet, dass** als inerte Trägerkörper Hohlzylinder eingesetzt werden.

12. Beschichtungskatalysator nach einem der vorstehenden Ansprüche, **dadurch gekennzeichnet, dass** die verwendeten Hohlzylinder eine der folgenden Größen aufweisen (Außendurchmesser x Höhe x Innendurchmesser):

```
6 x 5 x 3, 6 x 5 x 4, 6 x 4 x 4, 6 x 4 x 3, 5 x 5 x 3,
4 x 4 x 2, 4 x 4 x 1,5, 5 x 4 x 3, 5 x 5 x 2,5, 5 x 3 x 3, 4
x 3 x 1,5, 4 x 3 x 2, 3 x 3 x 1,5, 3 x 4 x 1,5 jeweils in
mm.
```

**13.** Beschichtungskatalysator nach einem der vorstehenden Ansprüche, **dadurch gekennzeichnet, dass** der inerte Trägerkörper ein Zylinder mit Durchtrittskanal und stirnseitlich nach außen abgeflachten Kanten darstellt.

**14.** Beschichtungskatalysator nach einem der vorstehenden Ansprüche, **dadurch gekennzeichnet, dass** der Aktivmassenanteil zwischen etwa 3 und 60 Gew.-%, bevorzugt zwischen etwa 3 und 50 Gew.-%, weiter bevorzugt zwischen etwa 5 und 40 Gew.-% liegt.

**15.** Beschichtungskatalysator nach einem der vorstehenden Ansprüche, **dadurch gekennzeichnet, dass** die Dicke der Beschichtung auf dem inerten Träger zwischen etwa 30 und 1000 $\mu$m, bevorzugt zwischen etwa 100 und 700 $\mu$m liegt.

**16.** Beschichtungskatalysator nach einem der vorstehenden Ansprüche, **dadurch gekennzeichnet, dass** der inerte Trägerkörper eine Porosität von weniger als 0,1 ml/g und vorzugsweise eine BET-Oberfläche von weniger als 1 $m^2$/g aufweist.

**17.** Beschichtungskatalysator nach einem der vorstehenden Ansprüche, **dadurch gekennzeichnet, dass** die weiteren metallischen bzw. metalloxidischen Komponenten der Komponente a) aus der Gruppe der Lanthaniden bzw. deren Oxiden ausgewählt sind.

**18.** Beschichtungskatalysator nach einem der vorstehenden Ansprüche, **dadurch gekennzeichnet, dass** die weiteren metallischen bzw. metalloxidischen Komponenten der Komponente a) ausgewählt sind aus Titan, Antimon, Zinn, Nickel, Chrom, Aluminium, Calcium, Magnesium, Vanadium, Niob, Silber und/oder Mangan.

**19.** Beschichtungskatalysator nach einem der vorstehenden Ansprüche, **dadurch gekennzeichnet, dass** das integrale Porenvolumen der Beschichtung nach Entfernen der organischen Anteile der Komponenten b) bzw. c) größer als 0,2, insbesondere größer als 0,25 ml/g liegt.

**20.** Verfahren zur Herstellung eines Beschichtungskatalysators gemäß einem der vorstehenden Ansprüche, insbesondere zur Oxidation von Methanol zu Formaldehyd, umfassend die folgenden Schritte:

> a) Bereitstellen eines inerten, vorzugsweise im wesentlichen unporösen Trägerkörpers,
> b) Herstellen einer wässrigen Suspension, enthaltend die Komponenten a), b), c) wie in den vorstehenden Ansprüchen definiert,
> c) Aufbringung der wässrigen Suspension gemäß Schritt b) auf den inerten Trägerkörper im Fließbettverfahren.

**21.** Verfahren nach Anspruch 19, **dadurch gekennzeichnet, dass** das Aufbringen der Beschichtung im Fließbettverfahren bei weniger als 100°C, vorzugsweise bei weniger als 80 °C, insbesondere bei weniger als 70 °C durchgeführt wird.

**22.** Verfahren nach einem der vorstehenden Ansprüche, **dadurch gekennzeichnet, dass** die Komponenten a) und b), vorzugsweise die Komponenten a), b) und c) in der wässrigen Suspension gemäß Schritt b) von Anspruch 15 als Suspension oder Dispersion und nicht in gelöster Form vorliegen.

**23.** Verfahren nach einem der vorstehenden Ansprüche, **dadurch gekennzeichnet, dass** der pH-Wert der wässrigen Suspension gemäß Schritt b) von Anspruch 20 bei Verwendung eines acetatstabilisierten $ZrO_2$-Sols zwischen etwa 3 und 5 liegt.

**24.** Verfahren nach einem der vorstehenden Ansprüche, **dadurch gekennzeichnet, dass** der pH-Wert der wässrigen Suspension gemäß Schritt b) von Anspruch 20 bei Verwendung eines salpetersäurestabilisierten $TiO_2$-Sols zwischen etwa 1 und 3 liegt.

25. Verfahren nach einem der vorstehenden Ansprüche, **dadurch gekennzeichnet, dass** der pH-Wert der wässrigen Suspension gemäß Schritt b) von Anspruch 20 bei Verwendung eines essigsäurestabilisierten $CeO_2$-Sols zwischen etwa 2 und 4 liegt.

26. Verfahren nach einem der vorstehenden Ansprüche, **dadurch gekennzeichnet, dass** die Komponente a) vor dem Auftragen auf den inerten Trägerkörper nicht auf über 200°C erhitzt wird, insbesondere nicht getempert bzw. calciniert wird.

27. Verfahren nach einem der vorstehenden Ansprüche, **dadurch gekennzeichnet, dass** die wässrige Suspension eine Teilchengröße ($D_{90}$) von weniger als 50 $\mu$m, bevorzugt weniger als 30 $\mu$m aufweist.

28. Verfahren nach einem der vorstehenden Ansprüche, **dadurch gekennzeichnet, dass** die Teilchengrößen der Komponenten a), b) und c) vor, während oder nach Herstellung der wässrigen Suspension durch einen Mahlvorgang eingestellt wird.

29. Verfahren nach einem der vorstehenden Ansprüche, **dadurch gekennzeichnet, dass** der Beschichtungskatalysator nach dem Aufbringen auf den inerten Trägerkörper auf eine Temperatur zwischen etwa 150 und 600°C, bevorzugt zwischen 200 und 550°C, weiter bevorzugt zwischen 250 und 500°C erhitzt wird.

30. Verfahren nach einem der vorstehenden Ansprüche, **dadurch gekennzeichnet, dass** das Erhitzen in einer Atmosphäre aus Luft, Inertgas und/oder Wasserdampf erfolgt.

31. Verfahren nach einem der vorstehenden Ansprüche, **dadurch gekennzeichnet, dass** das Erhitzen als Calcinierung bei einer Temperatur zwischen etwa 350 und 450°C, bevorzugt bei etwa 400°C erfolgt.

32. Verfahren nach einem der vorstehenden Ansprüche, **dadurch gekennzeichnet, dass** die Aufheizrate beim Erhitzen bzw. Calcinieren zwischen 0,1 und 10°C/min liegt.

33. Verfahren nach einem der vorstehenden Ansprüche, **dadurch gekennzeichnet, dass** der Wasserdampfgehalt beim Erhitzen bzw. Calcinieren mehr als 10 Vol.-%, bevorzugt zwischen 20 und 60 Vol.-% der verwendeten Atmosphäre beträgt.

34. Verfahren nach einem der vorstehenden Ansprüche, **dadurch gekennzeichnet, dass** das Erhitzen bzw. Calcinieren direkt im Reaktor in einem $O_2/N_2$-Gasstrom oder unter den für den Beschichtungskatalysator vorgesehenen Betriebsbedingungen stattfindet.

35. Katalysator, erhältlich nach einem Verfahren gemäß einem der vorstehenden Ansprüche.

36. Verwendung eines Katalysators gemäß einem der Ansprüche 1 bis 18 bzw. erhältlich nach einem Verfahren gemäß einem der Ansprüche 19 bis 31 zur Oxidation von Methanol zu Formaldehyd, insbesondere in einem Festbett.

37. Verwendung einer Kombination der Komponenten b) und c) wie in den vorstehenden Ansprüchen definiert zur Erhöhung der Abriebfestigkeit eines Beschichtungskatalysators.

**Figur 1**

Europäisches
Patentamt

**EUROPÄISCHER RECHERCHENBERICHT**

Nummer der Anmeldung

EP 05 02 5497

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 98/23371 A (CONSORTIUM FUER ELEKTROCHEMISCHE INDUSTRIE GMBH; RUEDINGER, CHRISTOPH;) 4. Juni 1998 (1998-06-04) * Seite 2, Zeile 12 - Zeile 18 * * Seite 4, Zeile 19 - Seite 5, Zeile 4 * * Seite 5, Zeile 31 - Seite 7, Zeile 37 * * Seite 9, Zeile 6 - Zeile 31 * * Seite 12, Zeile 21 - Zeile 30 * | 1-37 | B01J23/881 B01J35/02 B01J37/02 B01J37/08 C07C45/38 |
| Y | | 1-37 | |
| X | WO 99/62637 A (BASF AKTIENGESELLSCHAFT; HEIDEMANN, THOMAS; ROSOWSKI, FRANK; LINDEN, G) 9. Dezember 1999 (1999-12-09) * Zusammenfassung * * Seite 2, Zeile 19 - Seite 5, Zeile 17 * * Seite 12, Zeile 27 - Seite 13, Zeile 42 * | 1-37 | |
| Y | | 1-37 | |
| Y | US 5 217 936 A (SARUP ET AL) 8. Juni 1993 (1993-06-08) * Spalte 1, Zeile 48 - Zeile 67 * * Spalte 2, Zeilen 10-13 * * Spalte 2, Zeile 38 - Zeile 51 * | 1-37 | RECHERCHIERTE SACHGEBIETE (IPC) B01J C07C |
| A | EP 1 108 470 A (CONSORTIUM FUER ELEKTROCHEMISCHE INDUSTRIE GMBH) 20. Juni 2001 (2001-06-20) * Absätze [0004], [0005], [0016] - [0019], [0030], [0031] * | 1-37 | |
| A | WO 99/61433 A (BASF AKTIENGESELLSCHAFT; HEIDEMANN, THOMAS; WANJEK, HERBERT) 2. Dezember 1999 (1999-12-02) * Seite 4, Zeile 38 - Seite 6, Zeile 19 * * Ansprüche * | 1-37 | |

-/--

Der vorliegende Recherchenbericht wurde für alle Patentansprüche erstellt

| Recherchenort | Abschlußdatum der Recherche | Prüfer |
|---|---|---|
| München | 16. Dezember 2005 | Gosselin, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**Europäisches Patentamt**

## EUROPÄISCHER RECHERCHENBERICHT

Nummer der Anmeldung

EP 05 02 5497

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 10 2004 014918 A1 (BASF AG) 13. Oktober 2005 (2005-10-13) * Absätze [0026], [0027], [0035] - [0037] * ----- | 1-37 | |
| Y | WO 2005/037427 A (BASF AKTIENGESELLSCHAFT; HARTH, KLAUS; JOHANN, THORSTEN; WALSDORFF, CH) 28. April 2005 (2005-04-28) * Seite 1, Zeile 23 - Zeile 39 * * Seite 2, Zeile 22 - Seite 3, Zeile 7 * * Seite 4, Zeile 27 - Zeile 29 * ----- | 11-13 | |
| Y | EP 0 184 790 A (BASF AKTIENGESELLSCHAFT) 18. Juni 1986 (1986-06-18) * Seite 2, Zeile 18 - Zeile 27 * * Seite 3, Zeile 1 - Zeile 8 * * Seite 3, Zeile 30 - Zeile 43 * * Seite 4, Zeile 1 - Zeile 8 * ----- | 11-13 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 16. Dezember 2005 | Gosselin, D |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
................................................................................
& : Mitglied der gleichen Patentfamilie, übereinstimmendes
   Dokument

EPO FORM 1503 03.82 (P04C03)

# EP 1 792 651 A1

**ANHANG ZUM EUROPÄISCHEN RECHERCHENBERICHT**
**ÜBER DIE EUROPÄISCHE PATENTANMELDUNG NR.**   EP 05 02 5497

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-12-2005

| Im Recherchenbericht angeführtes Patentdokument | | Datum der Veröffentlichung | Mitglied(er) der Patentfamilie | | Datum der Veröffentlichung |
|---|---|---|---|---|---|
| WO 9823371 | A | 04-06-1998 | AT | 200989 T | 15-05-2001 |
| | | | BR | 9713454 A | 28-03-2000 |
| | | | CN | 1238714 A | 15-12-1999 |
| | | | DE | 19649426 A1 | 04-06-1998 |
| | | | EP | 0951351 A1 | 27-10-1999 |
| | | | ES | 2158609 T3 | 01-09-2001 |
| | | | JP | 2000505723 T | 16-05-2000 |
| | | | JP | 3416154 B2 | 16-06-2003 |
| | | | NO | 992553 A | 27-05-1999 |
| | | | TW | 394706 B | 21-06-2000 |
| | | | US | 6274763 B1 | 14-08-2001 |
| WO 9962637 | A | 09-12-1999 | CN | 1304336 A | 18-07-2001 |
| | | | DE | 19824532 A1 | 09-12-1999 |
| | | | EP | 1091806 A1 | 18-04-2001 |
| | | | ID | 28000 A | 03-05-2001 |
| | | | JP | 2002516751 T | 11-06-2002 |
| | | | KR | 2001052523 A | 25-06-2001 |
| | | | TW | 443944 B | 01-07-2001 |
| | | | US | 6528683 B1 | 04-03-2003 |
| US 5217936 | A | 08-06-1993 | KEINE | | |
| EP 1108470 | A | 20-06-2001 | AT | 220572 T | 15-08-2002 |
| | | | BR | 0005810 A | 02-01-2002 |
| | | | CN | 1298763 A | 13-06-2001 |
| | | | DE | 19959413 A1 | 21-06-2001 |
| | | | ES | 2179804 T3 | 01-02-2003 |
| | | | JP | 2001205107 A | 31-07-2001 |
| | | | NO | 20006216 A | 11-06-2001 |
| | | | US | 6624114 B1 | 23-09-2003 |
| WO 9961433 | A | 02-12-1999 | CN | 1131859 C | 24-12-2003 |
| | | | DE | 19823262 A1 | 02-12-1999 |
| | | | EP | 1084115 A1 | 21-03-2001 |
| | | | ES | 2197684 T3 | 01-01-2004 |
| | | | ID | 27092 A | 01-03-2001 |
| | | | JP | 2002516319 T | 04-06-2002 |
| | | | TW | 444004 B | 01-07-2001 |
| | | | US | 6700000 B1 | 02-03-2004 |
| DE 102004014918 A1 | | 13-10-2005 | WO | 2005092496 A1 | 06-10-2005 |
| WO 2005037427 | A | 28-04-2005 | KEINE | | |
| EP 0184790 | A | 18-06-1986 | DE | 3445289 A1 | 19-06-1986 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT
## ÜBER DIE EUROPÄISCHE PATENTANMELDUNG NR.

EP 05 02 5497

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-12-2005

| Im Recherchenbericht angeführtes Patentdokument | Datum der Veröffentlichung | Mitglied(er) der Patentfamilie | Datum der Veröffentlichung |
|---|---|---|---|
| EP 0184790 A | | JP 61141933 A<br>US 4656157 A | 28-06-1986<br>07-04-1987 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3978136 F
- US 3975302 A
- US 4181629 A
- EP 0068192 A
- EP 0294775 A
- EP 1127618 A1
- WO 2005037427 A1
- WO 2005011862 A
- DE 1280756 A

- DE 19828583 A
- DE 19709589 A
- DE 4006935 A1
- DE 10344845 A1
- WO 2005030388 A
- DE 10361517
- EP 0001570 A
- US 3852361 A

**In der Beschreibung aufgeführte Nicht-Patentliteratur**

- *J. Am. Chem. Soc.,* 1938, vol. 60, 309